Europäisches Patentamt

European Patent Office

Office européen des brevets

(11) Publication number: 0 171 927
A2

# EUROPEAN PATENT APPLICATION

(21) Application number: 85305000.3

(22) Date of filing: 12.07.85

(51) Int. Cl.⁴: A 61 B 5/02
A 61 B 5/00

(30) Priority: 13.07.84 JP 146376/84

(43) Date of publication of application:
19.02.86 Bulletin 86/8

(84) Designated Contracting States:
CH DE FR GB IT LI

(71) Applicant: SUMITOMO ELECTRIC INDUSTRIES LIMITED
No. 15, Kitahama 5-chome Higashi-ku
Osaka-shi Osaka 541(JP)

(72) Inventor: Yoneda, Kiwamu
c/o Osaka Works Sumitomo Electric Industries Ltd.
1-3, Shimaya 1-chome Konohana-ku Osaka(JP)

(72) Inventor: Kuwa, Kazuhiro
c/o Osaka Works Sumitomo Electric Industries Ltd.
1-3, Shimaya 1-chome Konohana-ku Osaka(JP)

(74) Representative: Rackham, Stephen Neil et al,
GILL JENNINGS & EVERY 53-64 Chancery Lane
London WC2A 1HN(GB)

(54) Patient monitor.

(57) A patient monitor (21) for monitoring body condition information such as blood $PO_2$/oxygen tension comprises means (2) for converting an electrical analogue signal representing body condition information detected by a sensor (1) into digital measurement value data at a predetermined time, means for taking (4) summary data out of the measurement values and storing (6) the summary data and means (4) for editing summary data stored in accordance with an output instruction and outputting (9) the edited summary data. Thus, the invention is useful in examining information on the condition of a body and leads to savings in the amount of recording paper used, as well as facilitating its storage.

./...

Fig. 1

SENSOR

L

2 — ANALOG INPUT DEVICE

3 — A/D CONVERTER

21

4 — CPU

5 — EPROM

10 — LCD-A

6 — CMOS RAM 61 / 62

11 — LCD-B

7 — REAL TIME CLOCK

12 — KEY INPUT

8 — SOUND GENERATOR   81   82

92

9 — DIGITAL OUTPUT

91 — ANALOG OUTPUT

14   13 — POWER SOURCE   AC

## "Patient Monitor"

This invention relates to medical appliances and more particularly, to patient monitors which can convert information on the state of a living body from human patients such as electrocardiogram, heart rate or blood $CO_2$/oxygen tension into electrical signals, store the electrical signals, process the electrical signals if necessary, edit the electrical signals and output them as summary data.

Prior art devices for measuring living body information on the condition of patients which are being observed by continuous monitoring of such faction as electrocardiogram, blood pressure or the like are designed instantly to output the measured values of such information and record the measured values on a recording paper or display them on a CRT (Cathode Ray Tube). A physician then examines the recorded or displayed values to monitor the condition of the patient.

The known method for instantly outputting measured data of such information and recording or displaying the measured data has, for example, the drawbacks enumerated hereinbelow and physicians have desired that the drawbacks inherent in this method be eliminated. The drawbacks are:

(a) The volume of paper on which the information is recorded becomes increasingly large as the measuring time elapses.

(b) Since data in wave form extend as a long line on the CRT or recording paper, it is difficult to pinpoint extraordinary or special points when seeking to diagnose.

(c) Storage of the information records is not easy.

According to this invention a patient monitor comprises

a sensor for detecting body condition information and converting the detected information into an electrical analogue signal;

means for converting the electrical analogue signal into digital measured value data and establishing summary data which is a collection of a plurality of representative data values deduced from the digital measured value data, the digital measured value data being provided by digitizing the electrical analogue signal at predetermined times dependent upon the type of the body condition information;

means for storing the summary data;

means for editing the stored data; and,

means for outputting the edited data in accordance with an outputting instruction.

The present invention provides a patient monitor which effectively eliminates the drawbacks inherent in conventional patient monitors and which edits information on the condition of a body in proper form and reduces it to summary data.

Another advantage of a particular embodiment of the present invention is to provide a patient monitor which can display or record such information in edited form. A further advantage of particular embodiment of the present invention is the provision of a patient monitor which is portable and serves as a data collection terminal unit.

The patient monitor of the present invention may incorporate means for displaying or recording (printing) the output of the edited data. Further, if necessary, the means for displaying or recording the edited data may be an external displaying or recording means which is connected to the patient monitor by means of a cable.

Furthermore, the patient monitor of the present invention may be designed as a portable device by incorporating a battery power source whereby the monitor can function as a data terminal unit for collection of information on the condition of a body.

A particular embodiment of the present invention will now be described with reference to the accompanying drawings; in which:-

Figure 1 is a block diagram of the monitor;

Figure 2 is a perspective view of the patient monitor together with an external recorder;

Figure 3 is a block diagram of the external recorder; and,

Figure 4 is a view showing one example of summary data for a patient printed out by the external recorder.

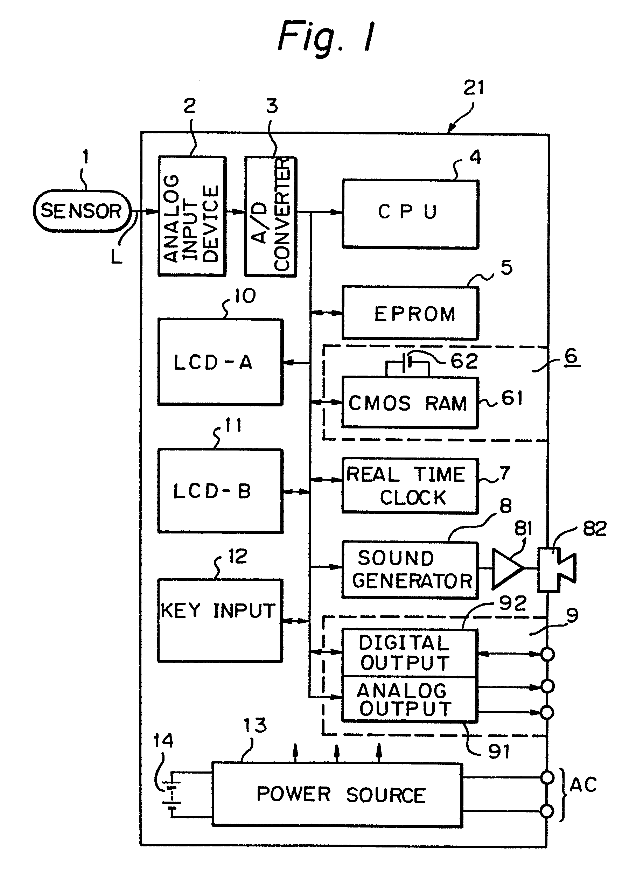

One preferred embodiment of the patient monitor according to the present invention will now be described. Figure 1 is a diagram of the system of the principal components of the patient monitor embodying the present invention. In Figure 1, a sensor 1 attached to a patient (not shown) detects an item of information such as blood $CO_2$/oxygen tension and the detected information is fed as an electrical analogue signal through a lead L to an analogue input device 2. The analogue input device 2 amplifies the analogue signal and feeds the amplified analogue signal to an A/D (analogue/digital) converter 3. The A/D converter 3 converts the electrical analogue signal into an electrical digital signal each time the converter receives a sampling signal from a CPU 4 and feeds the electrical digital signal to the CPU 4. An EPROM (erasable and programmable ROM) 5 stores a program for controlling the entire system shown in Figure 1. A memory 6 consists of a CMOS RAM 61 and a lithium cell 62 for baking the RAM 61 up. The RAM 61 is provided with an area where summary data transferred from the CPU 4 are stored, an area where parameters input thereto from a key input 12 and adapted to convert the data form of the digital signal into body condition information data are stored and an area where ID (Index Data) are stored. The RAM 61 and CPU 4 reciprocate data therebetween. A real time clock circuit 7 outputs real time information relating to day, hour, minute and second and a timing signal to the CPU 4. The CPU 4 outputs a sampling signal at a predetermined time interval Ti based on a timing signal transmitted from the real time clock circuit 7 and obtains an electrical digital signal at the time interval from the A/D converter 3. Furthermore, the CPU 4 reads parameters corresponding to body condition information being monitored out of the RAM 61 and converts the data form of the digital signal into a measured value data in accordance with the parameters. A representative value is selected as a "summary data" from the converted data (measured value data).

The selection of the above-mentioned summary data may be performed by extracting measured value data as summary data at a time interval $n$ x Ti or extracting measured value data at a time interval Ti and then averaging $n$ measured value data to obtain single summary data. The time interval Ti, number of measured value data $n$ and data extraction procedures referred to above are selectively and suitably employed in dependence upon the type of body condition data to be monitored. For example, for monitoring blood $PO_2$/oxygen tension, summary data are extracted at a time internal of $n$ x Ti = 10 sec. under the conditions of Ti = 0.5 sec. and $n$ = 20. In this case, when one data is expressed by 16 bits, in order to store summary data corresponding to 8-consecutive hours' measurement, a capacity capable of storing over 5,760 bytes is sufficient.

The CPU 4 feeds the measured value data in succession to a measured value display (LCD-A) 10 which displays the same data. The measured value display 10 is formed of liquid crystal and has a display capacity of 7 segments x 4. Furthermore, the CPU 4 transfers summary data obtained from measured value data in a predetermined number to the RAM 61 which stores the summary data together with other data like time data representing the time when the summary data were extracted. When the obtained measured value data exhibit critical values, the CPU 4 discriminates such values and outputs an alarm signal to a sound generator 8 which in turn outputs an alarm through an amplifier 81 to an alarm speaker 82 which produces an acoustic alarm. Simultaneously, the CPU 4 transfers a danger message from the EPROM 5 to a message display (LCD-B) 11 which displays the danger message.

The message display 11 is formed of liquid crystal and has the display capacity of 16 letters x 2 lines to serve as an auxiliary unit of the display 10.

The key input 12 has the function of presetting the above-mentioned parameters to the RAM 61as well as an additional function of instructing the RAM 61 to output summary data stored therein. A power source 13 is supplied with AC power from an external power supply and rectifies AC voltage to DC voltage with which a cell 14 is charged. The power source 13 also supplies necessary power to the components constituting the system. The cell 14 serves as the battery power source. It is to be noted that the system shown in Fig. 1 is constructed to be portable and can be effectively used as a data collection terminal unit.

Upon receiving an output demanding signal from the key input 12, the CPU 4 reads out summary data stored in the RAM 61. The CPU 4 edits summary data input thereto in a form, such as frequency distribution data, which can be easily read by a person monitoring it, for example, a physician. Furthermore, in order to adapt the summary data output to a predetermined communication procedure, the CPU 4 converts the output form and outputs the converted summary data into the digital output 92 of an output region 9 with a predetermined timing determined by the CPU 4. Simuntaneously, the CPU 4 also edits additional information such as the days and times when summary data were sampled, ID relating to a patient and the like and converts the output form of them to be output to the digital output 92. The digital output 92 is a data transmission system of international standard and consists of a digital interface RS-232C. The output region 9 also has an analog output 91 which is adapted to be connected to a pen recorder or the like which requires an analog signal input. That is, when the analog output 91 is provided with measured value data or summary data from the CPU 4, the analog output 91 converts the data from digital into analog and outputs them as an analog signal.

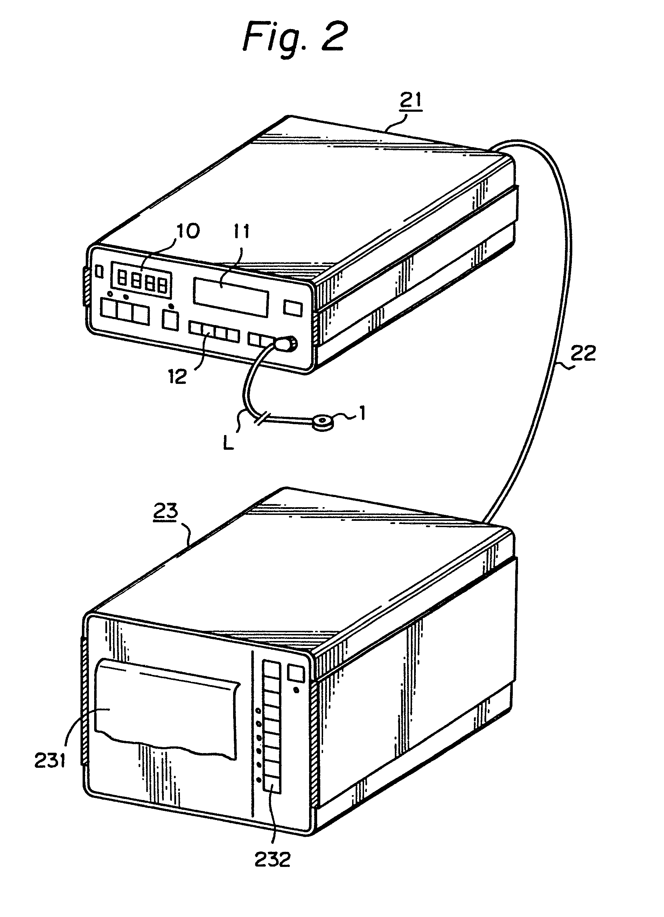

Fig. 2 is a perspective view of the patient monitor embodying the present invention in which the main body 21 encircled by the solid line, the sensor 1, lead L, measurement display 10, message display 11 and key input 12 are indicated with numbers which correspond to those shown in Fig. 1. The output terminal of the digital output 92 shown in Fig. 1 is connected to a summary output exclusive recorder 23 by means of a cable 22. In Fig. 2, reference numeral 231 denotes a graphic printer and reference numeral 232 denotes a key input.

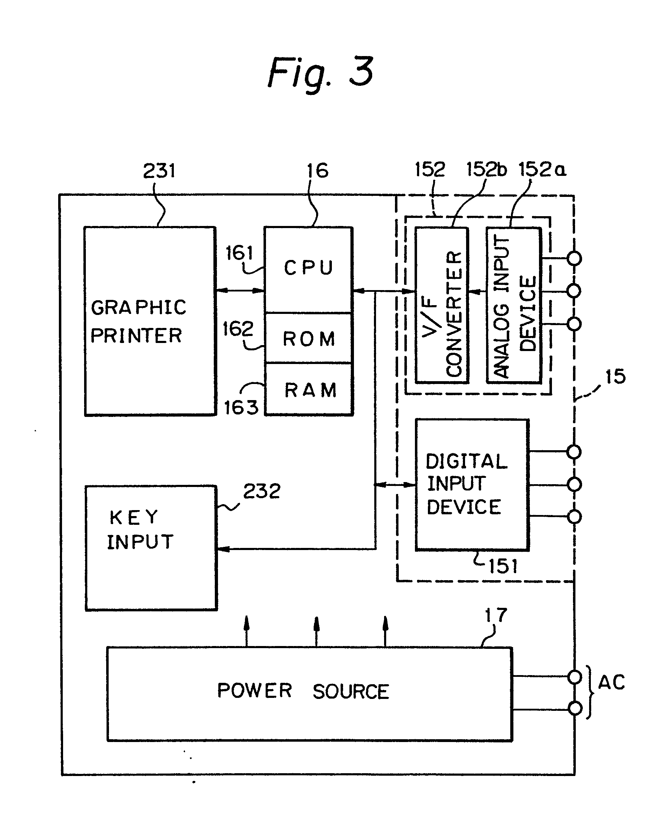

Fig. 3 shows a diagram of the system of the principal components of the recorder 23 and the cable 22 is connected to the input terminal of a digital input 151 of the input region 15. The digital input 151 consists of a digital interface RS-232C. Summary data input to the digital input 151 are fed to a control 16. The input region 15 is provided with an analog interface 152 to process an analog input. The analog interface 152 consists of an analog input 152a and a V/F (voltage/frequency) converter 152b and analog data input to the input 152 are converted into digital data which are fed to the control 16.

The control 16 consists of a CPU 161, a ROM 162 and a RAM 163. Summary data fed to the control 16 are processed in accordance with the instructions from the key input 232 in the CPU 161 and output to the graphic printer 231. The graphic printer 231 employs a stepping motor whereby printing and paper feed are performed by predetermined amounts at predetermined time intervals in accordance with control signals from the CPU 161. Assuming that printing is performed by one step per summary data, the printer can be controlled quite simply. In such a case, when summary data taken from the sampling time of 20 seconds are output to the graphic printer of 1/6 mm printing step, data produced during a period of 1 hour can be recorded on a chart of 30 mm. The graphic printer 231 is preferably a thermal graphic printer because the thermal graphic printer performs printing very quietely. The components of the recorder 23 are supplied with a necessary amount of power from the power source 17 which rectifies mains AC.

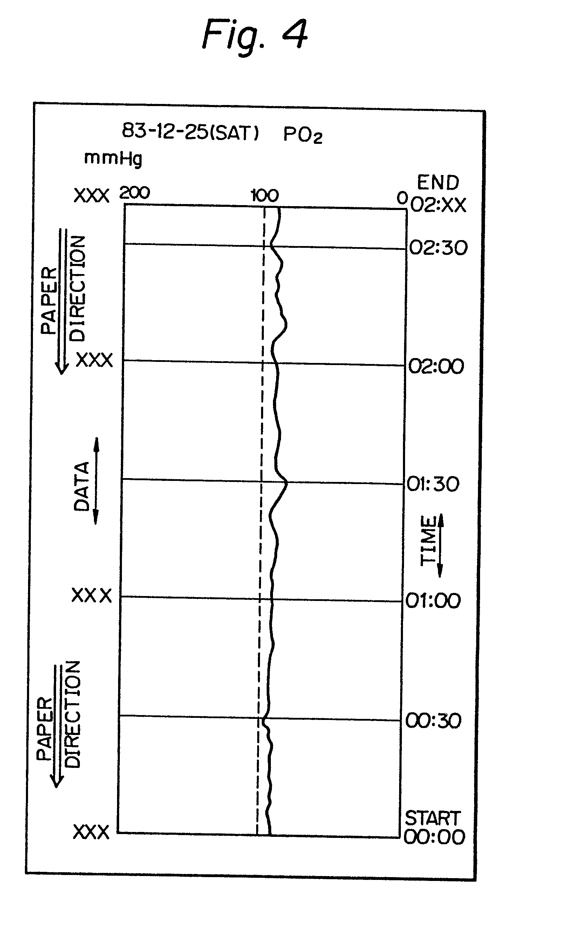

Fig. 4 shows an example in which thermal data relating to blood $CO_2$/oxygen tension are printed by the recorder 23 as body condition information.

When the patient monitor embodying the present invention is employed, since data are extracted from body condition information which is produced successively and are stored and output, various items of body condition information can be compared with each other and examined, and data occurring over a long period of monitoring can be edited and taken out. Furthermore, it is also possible where necessary to pick only important portions out of successive recording data and then to examine these fully. Additionally, savings in the amount of recording paper used can be attained and storing of the paper is made very easy.

In the illustrated embodiment, although the patient monitor is a separate type in which the monitor main body 21 is connected to the recorder 23 by means of the cable 22, it is also contemplated by the present invention, that the monitor main body 21 of Fig. 1 and the recorder 23 of Fig. 3 may be housed in a common case.

-8-

0171927

## CLAIMS

1. A patient monitor (21) for monitoring body condition information comprising:

a sensor (1) for detecting body condition information and converting the detected information into an electrical analogue signal;

means (2, 3, 4) for converting the electrical analgoue signal into digital measured value data and establishing summary data which is a collection of a plurality of representative data values deduced from the digital measured value data, the digital measured value data being provided by digitizing the electrical analogue signal at a predetermined time dependent upon the type of the body condition information;

means (6) for storing the summary data;

means (4) for editing the stored data; and,

means (9) for outputting the edited data in accordance with an outputting instruction.

2. A patient monitor according to claim 1, in which the means (4) for seeking the summary data includes means (4) for processing the measured values and seeking an average of the measured values.

3. A patient monitor according to claim 1 or claim 2, further including means for recording (231) or displaying (19) the edited data output from the output means (9).

4. A patient monitor according to claim 3, in which the means for recording (231) or displaying (19) the edited data is connected to the output means (9) by a cable (22).

5. A patient monitor according to claim 1, in which the monitor (21) includes a battery power source (13) whereby the monitor (21) is made portable.

# Fig. 1

# Fig. 2

# Fig. 3

# Fig. 4